(19) Europäisches Patentamt / European Patent Office / Office européen des brevets

(11) **EP 1 873 241 A1**

(12) **EUROPEAN PATENT APPLICATION**

(43) Date of publication:
**02.01.2008 Bulletin 2008/01**

(51) Int Cl.:
***C12N 15/10*** *(2006.01)*

(21) Application number: **06013576.1**

(22) Date of filing: **30.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Qiagen AS**
**0884 Oslo (NO)**

(72) Inventors:
- **Kamfjord, Dyre**
  **1392 Vettre (NO)**
- **Skagestad, Vidar**
  **1344 Haslum (NO)**

(74) Representative: **Polypatent**
**Postfach 40 02 43**
**51410 Bergisch Gladbach (DE)**

(54) **Process for isolating nucleic acids**

(57) The present invention relates to a process for isolating total nucleic acid from a nucleic acid-containing sample and a kit for carrying out said process. More specifically, it relates to the isolation of RNA from a nucleic-acid containing sample.

EP 1 873 241 A1

**Description**

The present invention relates to a process for isolating total nucleic acid from a nucleic acid-containing sample and a kit for carrying out said process. More specifically, it relates to the isolation of RNA from a nucleic-acid containing sample.

Procedures involving nucleic acids such as DNA and RNA continue to play a crucial role in biotechnology. Early methods of isolating nucleic acids involved series of extractions with organic solvents, involving ethanol precipitation (Amersham, US Patent 5 681 946) and dialysis of the nucleic acids. These methods are relatively laborious and often result in low yield.

Later methods take advantage of the fact that nucleic acids are bound to silica surfaces in the presence of chaotropic salt solutions. This was originally described for diatomaceous earth and for silicon dioxide particles (Boom et al, US Patent 5 234 809). More recently, the use of magnetically susceptible particles with silica surfaces have been described (Promega, US Patent 6 027 945, Toyobo, US Patent 6 582 922, on paramagnetic silica particles, WO 03/84976, on ferri magnetic silica particles), making the practical handling of the nucleic acid isolation system easier. Alternatively, a combination of chaotropes and alcohols can be used (QIAGEN, WO 95/01359).

Numerous methods exist for the isolation of RNA. Because of the presence and stability of RNAses in biological material, it is very important that lysis of any sample and RNAse inactivation occur almost simultaneously. The classical method utilizes a mixed buffer of salt and organics (i.e., sodium acetate phenol/chloroform) in order to lyse sample and deactivate RNAses. And this is still by far the method of choice in respect to number of use, despite its hazardous chemicals.

Alternatively, a strong detergent (for instance SDS) has been utilized for the lysis and RNAse deactivation. Heat et al. (US 5 973 137) describe the use of a lysis solution comprising a low pH, an anionic detergent and a chelating agent in the absence of chaotropes, and in combination with a centrifugation/phase separation purification procedure.

Yet another alternative arises when the sample is lysed under chaotropic condition/influence, a method which found widespread use. The chaotrope has excellent properties to simultaneously lyse the sample and to deactivate the RNAse activity. In addition, chaotropes induce nucleic acid binding to any silica surfaces present (Boom, et.al).

It has been shown that the pH of the chaotrope based lysate can have a crucial influence on RNA preparations. Isolation of RNA under acidified chaotrope conditions generally gives a higher RNA (over DNA) selectivity (Noonberg et.al; BioTechniques 19, 731-733 (1995)). However, in conflict with the present invention and one further (US 5 155 018), Chomczynski teaches that pH should not be lower than 4 (US 4 843 155).

Isolation of RNA under acidified conditions (pH < 6) is for instance described by Gillespie (US 5 155 018). Although extraction at low pH favours RNA binding over DNA, DNA is still present in the final nucleic acid preparation sample. Adding lithium salt to the acidified lysate has been found to favour RNA binding over DNA even more. Kuroita (US 5 990 302), utilized the chemical property of lithium ions to insolubilize aqueous ribonucleic acids (Molecular Cloning, 2nd ed., 1.40 (1989)). Kuroita also mentions the use of non alcoholic, low salt aqueous washing buffers.

Kuroita and Gillespie describe the final elution (which corresponds to dissociation of nucleic acids from the solid phase into solution) of nucleic acids by applying either heat or increasing the pH in the elution buffer.

The experimental set-up for the present method partly is based on teaching from the literature, pointing to the possibility of RNA preparation at acidified pH, under chaotropic conditions and with solid phase extraction which can be carried out completely without organic solvents. This is of course very beneficial since organic solvents (e.g. alcohol) left in the final elute/nucleic acid product often imposes trouble to downstream analysis.

In spite of the advantages reached by using a combination of chaotropic lysis and binding, and solid phase separations for RNA preparations, typically processing of a high sample input load (exemplified by but not restricted to cultured cells) requires an initial disruption of the sample material. That is e.g. breaking of huge DNA, DNA-DNA, DNA-RNA, DNA-protein or RNA-protein complexes to reduce the viscosity of the lysate (It is well known to those skilled in the art that RNA binding suffers from viscous binding media). If this is not done the overall yield of isolated nucleic acids, and in particular of RNA, will decrease considerably, both when lysed under neutral or acidified chaotropic conditions.

This effect can partly be overcome by increasing the amount of solid phase employed (to enhance the RNA kinetics in a viscous binding media), or by increasing considerably the overall binding time (same reason as above). That means, for an efficient binding of RNA from high sample input using the prior art methods of choice, a high solid phase concentration is needed along with long binding time. But at high sample input, even without sample disruption, a further risk exists, which is clogging/clumping of the solid surface employed and thus even lower overall yield. The clogging/clumping is most likely due to DNA molecules serving as bridging points between solid phase binding sites.

Not only the yield, but also the quality of isolated nucleic acids suffers since clogging/clumping of the solid phase allows less efficient washing(s) to remove potential contaminants from the solid-nucleic acid complexes.

And finally a higher sample input eventually leads to a higher input of DNA, (those skilled in the art will realize that the DNA content is linear dependent on the sample input) and the relative overall binding of RNA then becomes even worse.

To summarize, the disadvantages of the prior art on high sample input are

- necessity of a cumbrous physical (for instance by means of a syringe, rotor mixer or similar) or enzymatic (for instance by use of Proteinase K) pretreatment sample disruption step
- long binding times as lysate becomes viscous
- solid phase induced clumping/clogging, which creates less efficient washing, and unsuitable for e.g. automation
- low yield of output RNA per sample input and per solid phase employed

The reason for these observations most likely relates to DNA as a) a bridging molecule between solid surface moieties inducing clumping of those and b) formation of huge DNA-DNA, DNA-protein, DNA-RNA complexes, either binding to the solid phase or resulting in high lysate viscosity, overall hindering/reducing the ability for RNA to bind.

The present invention addresses these disadvantages of the prior art, in particular those from high load sample inputs.

Thus, the object of the present invention was to provide an efficient, quick and easy method for preparation of nucleic acid from nucleic acid containing samples.

This object is met by a process for isolating nucleic acids from a nucleic acid containing sample, comprising contacting a nucleic acid containing acidified lysate with a nucleic acid binding solid surface, wherein the lysate comprises further at least the following components:

a) a chaotropic salt,
b) an organic, water soluble acidic polymer.

As used herein the "nucleic acid containing sample" is any sample containing nucleic acid sequences like DNA or RNA. As a non-limiting example such samples can have their origin from tissue or cells, and can be provided either as whole tissue or cells, or they can be already disrupted, or samples can be e.g. from sperm or from plant material. Further samples comprising dissolved (isolated) nucleic acids fall under the definition of nucleic acid containing sample.

The term "lysate" is herein used for any liquid sample, comprising nucleic acids obtainable from a nucleic acid containing sample. Preferably the lysate is obtainable by lysis or disruption of cells or tissue, however, any sample comprising "free" nucleic acids (outside of a closed cell) according to the present invention falls under the definition "lysate".

A lysate can be regarded as "acidified" when the pH of the lysate (as a whole) is pH 6 or less. Typically the pH is in the range from 1 to 6, preferably from 2 to 4, more preferred from 2 to 3.

A "chaotrope solution" means a solution containing a chaotropic salt. "Chaotropic salt(s)" are known to persons skilled in the art, e.g. usable for protein precipitation. Preferred chaotropic salts are described in detail later in this description.

A nucleic acid binding solid surface as used herein means any solid surface capable to bind nucleic acids. Details of suitable surfaces see below.

Accordingly, in a first aspect in one embodiment the present invention provides a process for isolating nucleic acids from a nucleic acid-containing sample, comprising:

(a) providing an acidified chaotrope lysis solution;
(b) providing a nucleic acid binding solid phase capable of binding nucleic acid in the presence of the chaotrope;
(c) providing an organic, water soluble acidic polymer;
(d) contacting the lysate with the nucleic acid binding solid phase in a liquid phase comprising the polymer; and
(e) optionally separating the solid phase with the nucleic acid bound thereto from the liquid phase

The inventive process further can comprise at least one of the following additional step(s): washing, purification, nuclease treatment (optionally) and elution(s), for example washing of the particle/nucleic acid complex with chaotropic substance containing washing buffer, washing of the particle/nucleic acid complex with aqueous washing buffer, elution of the nucleic acids into aqueous buffers for enzyme treatment of the isolated nucleic acids, rebinding of isolated nucleic acids to a solid phase (beads, columns or membranes) after enzymatic DNAse treatment, any washing steps that are relevant to the solid phase used for rebinding, and final elution into an aqueous buffer for further use of the isolated nucleic acids.

In a second aspect, the present invention provides a kit for isolating nucleic acids from a nucleic acid-containing sample, which kit comprises:

(a) a chaotropic salt;
(b) a nucleic acid binding solid phase capable of binding nucleic acid in the presence of the chaotrope; and

(c) a water soluble, acid organic polymer.

The kit can comprise the mentioned material either as powder material for preparation of solutions or dispersions, respectively, or as "ready-to-use" prepared solutions / dispersions.

It has surprisingly been found that presence of certain acid polymers e.g. poly(carboxylic acids), exemplified but not restricted to, poly(acrylic acids), in presence of acidified chaotropes and when contacted with a solid nucleic acid binding phase, has the following positive effects on the isolation of nucleic acid from a nucleic acid containing sample material:

- it renders a physical or enzymatic disruption of the sample unnecessary,
- it fully removes clumping of the solid phase,
- it allows the input of high content sample with a corresponding linear increase in yield of isolated nucleic acids
- it strongly reduces the total binding times needed,
- it allows a considerable reduction in solid phase employed per out-put of nucleic acid,

compared to approaches in which such a polymer is absent (like in prior art).

Using the method of the present invention the isolation of RNA from for instance 30 million collected cells is not only doable, but also very conveniently performed, when the sample is lysed under acidified (for example pH 2.5) chaotrope conditions, and then bound to surfaces like silica surfaces under the influence of a e.g. poly(acrylic acid) or salt thereof. It was found that the yield of nucleic acid from HeLa cultured cells is independent from a former disruption (see Experiment 3). Furthermore, regardless of the amount of sample input employed, no clumping of the solid surface during the isolation steps (see Experiment 1) was found. A dramatic reduction of the amount of employed silica solid phase per quantity of isolated nucleic acids (from 20 to 2.5 mg, see Experiment 6) was possible. Finally, it is possible to dramatically reduce the binding time compared to the prior art (from minutes to max 10 sec, see Experiment 1 and 4B).

The viscosity of the lysate (comprising chaotropes + nucleic acid containing sample + polymer) in the method of this invention can be strongly reduced, compared to cases in which (an) according polymer(s) is absent. This can already be seen by a visual inspection. In that respect, the poly (acrylic acids) might have the same effect as a physical disruption and can be seen as a chemical alternative to physical or enzymatic disruption.

Without wishing to be bound to the following theory, it is speculated that these carboxylic polymers strongly interact with the DNA's of the sample in the lysate, maybe by forming polymer-DNA complexes, reducing the DNA molecules extension, and thus reducing the viscosity of the lysate. The interaction probably is related to the ability of these polymers (on basis of $RCO_2\text{-}H \rightarrow RCO_2^- \ H^+$) to interact in H-bindings of the nucleic acids. Addition of 30% EtOH to the lysate, still in the presence of polymer, gave strength to this hypothesis, since in this case, no effect was found from the polymers and the performance and yield of nucleic acid resembled the prior art, pointing towards an EtOH assisted blocking of the H donor/ H acceptor polymer-nucleic acid association(s).

However, excluding alcohols is not part of this invention, which means that alcohols might still be used as part of the lysate, in binding or in washing steps, at concentrations developed by those skilled in the art.

Based on prior art teaching one might have expected that the presence of a detergent in the lysate would show the same strong effect as the acid polymer used according to the present invention. An experiment wherein the poly (acrylic acid) was replaced by e.g. Triton X-100 in the lysate showed a surprisingly weak performance compared to the preferred embodiment of this invention. However, a lysate comprising both, a detergent (exemplified by Triton X-100) AND an acid polymer such as poly(acrylic acid) turned out to be one of the preferred embodiments of the present invention. Those skilled in the art will recognise any detergent(s) soluble in the lysis buffer or the lysate (that is after the nucleic acid containing sample was lysed) of this invention (in the presence of the polymer), as serving according to the invention. However, use of a detergent might include problems in respect to foaming of sample, and thus make handling less easy. Therefore, the most preferred embodiment of this invention avoids the use of detergents. In case a detergent is contained in the lysate its concentration preferably ranges from 0,01 to 5 % (v/v), more preferred from 0,1 to 3 % (v/v) and most preferred from 0,3 to 1 % (v/v).

After capturing DNA somehow by the polymers, RNA strands of the sample are quite free to compete and bind to the solid phase with a high efficiency (kinetic's), much more than when this binding is strongly hindered by DNA competition, in the absence of polymers and under high viscosity conditions. Those skilled in the art will be aware of RNA suffering from binding to a solid phase under high viscosity conditions (that's the reason why DNA usually is disrupted and broken down). Using higher sample content, more DNA will compete with RNA for the same solid phase binding sites. And if, as it is, the binding of RNA is hindered by the presence of DNA, this will strongly hinder binding of RNA from high sample input load, despite addition of more solid phases.

Without intended to being bound thereto the basis of this invention can be explained by equilibrium theory:

## DNA + RNA + Solid Phase = DNA-RNA-Solid Phase

Solid phase is of equal importance, and thus for simplicity can be removed from equation, resulting in

$$[RNA]_{isolated} = [DNA]_{lysate}[RNA]_{lysate}/[DNA]_{isolated}$$

If now $[DNA]_{lysate}$ is strongly reduced by $[DNA]_{lysate}$ being bound to the polymer of this invention, and $[DNA]_{isolated}$ is still constant (see below), equilibrium shifts towards more $[RNA]_{isolated}$.

Interestingly, the overall binding of DNA ($[DNA]_{isolated}$ in the equation above) in the presence of the polymer is just as good as in its absence. Thus, generally the polymer strongly enhances the RNA kinetics compared to DNA, although the thermodynamic of the systems is virtually unchanged.

Those skilled in the art will realize that a (complete) removal of DNA is possible in line with this invention, preferably by introducing a DNA digestion step after the first lysis and binding step. Removing DNA prior to or as part of the lysis and binding steps (e.g. with DNAse), will however resemble what has been referred to as prior art enzymatic degradation of the lysate. Of course, such degradation can be carried out within the scope of this invention, if the acidic polymer is present during subsequent binding to the solid phase.

The nucleic acid containing sample of this invention typically comprises a biological sample such as a cellular sample. In general, samples such as tissues are also applicable to this invention. In latter case an additional mechanical or enzymatic step to disrupt the tissue can be required prior to binding of nucleic acids to nucleic acid binding solid phases, as it is known in the art.

The nucleic acid to be isolated may be RNA, DNA or a modified form thereof. When the nucleic acid is RNA, this may be rRNA, mRNA or total RNA.

The chaotropes usable according to the invention generally comprise a chaotropic ion, provided at a concentration which is sufficient to cause the nucleic acid to loose its secondary structure and, in case of double stranded nucleic acids, to melt. The chaotropes preferably comprise a guanidinium salt, urea, or sodium or potassium iodide, chlorate, perchlorate or (iso)thiocyanate or any mixtures thereof. Preferred chaotropes include guanidinium salts free of thiocyanates (to avoid development of HCl under acidified conditions).

A typical range for concentration of chaotropes for nucleic acid silica binding is 2 to 6 M of chaotropic salts, however, any suitable concentration resulting in the isolation of nucleic acids should be considered as being according to the invention.

The nucleic acid binding solid phase must be capable of binding nucleic acids in the presence of a chaotrope, but is not limited to any specific material. Various material are known as nucleic acid binding solid phases, including silica based material such as those described in US 5234809, polymeric material including latex and polystyrene-based material such as those described in WO96/18731. In general, any solid surface materials known to bind nucleic acid under the influence of a chaotrope will be applicable to this invention.

The form of the solid phase includes sheets, sieves, sinters, webs, membranes, beads and fibres. Particles are particularly useful as these may be packed in a column or used in suspension. Magnetic particles are particularly beneficial because of the ease with which they can be separated from an associated liquid phase in a magnetic field. Silica-based magnetic particles usable in the present invention include those described in WO 03/084976 (silica coated magnetic particles).

The acidic polymer used according to this invention can be any acidic polymer, preferably a poly(acrylic acid) or poly (methacrylic acid). However, this invention is not restricted to poly(acrylic acids), nor is it restricted with regard to their molecular weight. From the experiments, it is evident that a broad range of molecular weights can be used (from $M_w$ 2000 to 200 000). The concentration of the used polymers (weight of polymer / volume of lysate= w/v (wt%)) can also be within a very broad range, which is from $1*10^{-5}$ to less than 50 wt%, more preferably from 0.05 to 5 wt%, most preferably from 0.1 to 2.5 wt%. The examples show for instance the use of a poly(acrylic acid) of MW 2.100, ranging from 0.14 wt% to 2.2 wt% in the lysate.

The use of poly(carboxylic acid) is a preferred embodiment of this invention. They are very convenient to use, partly due to their ease of access, low toxicity and high solubility in water. Those skilled in the art are aware of opportunities to select dedicated poly(carboxylic acid) polymers, that is the polymer should contain acidic functionalities, which also can be present as their corresponding salts. Other organic acidic polymers, for instance such containing sulphuric or phosphorous atoms, like polyphosphoric acid alkyl esters, or mixtures of such polymers may also be applicable to this invention. Further copolymers containing any combination of carboxylic acid, sulfonic acid or alkylphosphoric acid monomers are usable.

Except that the polymer preferably is present when the sample is contacted with the nucleic acid binding solid phase, there is no particular limitation on how the polymer should be provided. Conveniently, the polymers can be provided as free acids, as salts, or as a combination thereof, or as a mixture of chemical different carboxyl polymers, added separately, or provided with the chaotropes, the solid phase or even the sample. A potential advantage arises if the polymer is introduced to the lysate, after the sample has been contacted and lysed by the chaotropes. The chaotrope solution can be acidic or neutral, although acidified lysis is preferred. Highly preferred according to this invention is that the overall pH of the final lysate is < 6 when the binding to the solid phase is due. Thus, although not very favourable, chaotropes, samples and nucleic acid binding solid phase can be contacted prior to the addition of a polymer.

The step of separating the solid phase with the nucleic acid bound thereto from the liquid phase is generally recommended in order to remove contaminants in the liquid phase. Further washing steps may be applied to the solid phase. Any convenient separation step known to those skilled in the art can be utilised.

For washing one approach, as indicated by Kuroita et al, might be the use of the following buffers:

(a) Wash I 2-6 M GuHCl, (b) Wash II and III water or 10mM Tris.

When a magnetic solid phase is used, separation is simplified by being carried out in the presence of a magnetic field. When a membrane or non magnetic particles are used, separation can be carried out via centrifugation.

Depending on the mode in which the isolated nucleic acid is required, a further elution step can be provided. If the nucleic acid shall be eluted from the solid phase, applying an elution buffer under elevated temperatures can be beneficial. In the experimental part, the elution typically is achieved in 10mM Tris pH 8 at 65°C for 3 min. Alternatively, increasing the pH of the elution buffer will induce elution even in the absence of heat, as can be seen from the experiments in which it was eluted cold at pH 11.5 (see examples).

Figures:

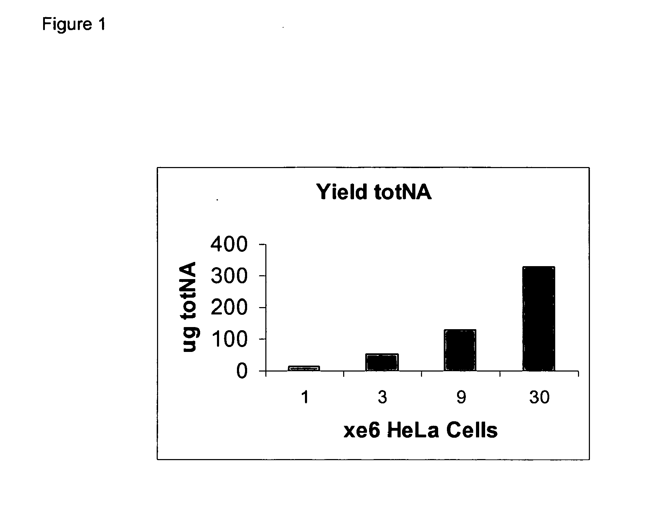

Figure 1: Yield (in $\mu$g total nucleic acid) from different input amounts of HeLa culture cells as prepared according to experiment 1, measured on a Spectrophotometer (Perkin Elmer, lambda EZ 201) with the 260/280 ratio ranging $\pm$2.1, indicating a mixture of RNA and DNA. Elution volume 200 $\mu$l.

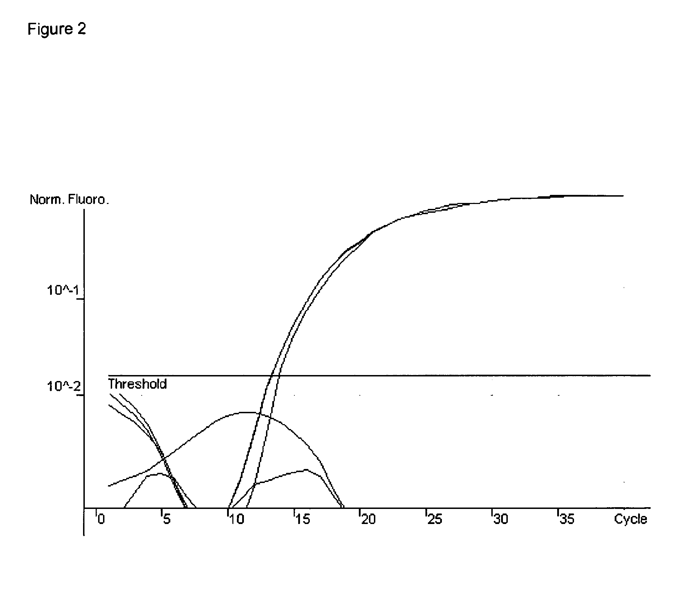

Figure 2: Plot of a real time PCR sample of 5 $\mu$l of a 200 $\mu$l elution volume, utilizing the eluate from 30 million HeLa cells (see Figure 1) of nucleic acids isolated according to procedure of experiment 1, transferred into a 25 $\mu$l QIAGEN QuantiTect B-actin qPCR kit sample, investigated on a Corbet Real-Time PCR instrument.

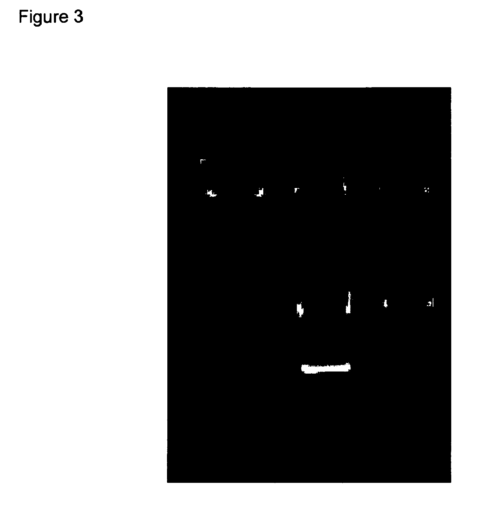

Figure 3: 0.8 % agarose gel showing 5 $\mu$l each of a 200 $\mu$l elution volume of the nucleic acids isolated according to experiment 2, using different molecular weights of the polymer. (a) nucleic acid isolated using poly(acrylic acid) MW 15.000, (b) nucleic acid isolated using poly(acrylic acid) MW 2.100, (c) nucleic acid isolated using poly(acrylic acid) MW 200.000.

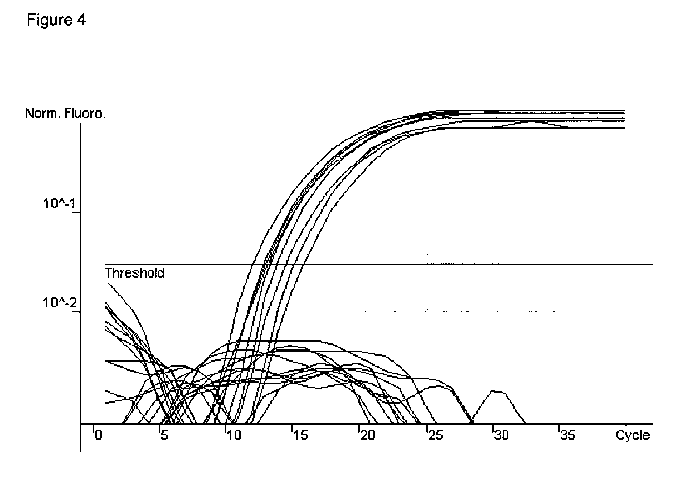

Figure 4: Plot of a real time PCR sample of 5 $\mu$l each of a 200 $\mu$l elution volume, utilizing the eluate of experiment 2, part (a), (b) or (c), respectively (see Figure 3), transferred into 25 $\mu$l QIAGEN QuantiTect B-actin qPCR kit samples, investigated on a Corbet Real-Time PCR instrument.

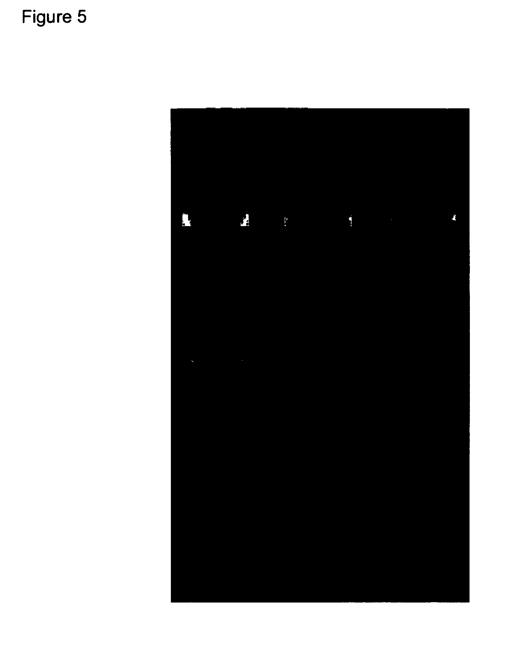

Figure 5: 0.8 % agarose gel showing 5 $\mu$l each of a 200 $\mu$l elution volume of the nucleic acids isolated according to experiment 3, using different concentrations of a polymer (MW 2.100). (a) 20 $\mu$l of 10 % polymer solution added, (b) 50 $\mu$l of 10 % polymer solution added, (c) 200 $\mu$l of 10 % polymer solution added.

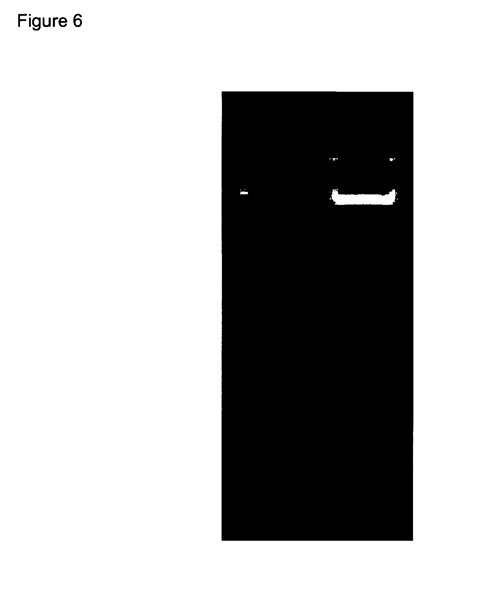

Figure 6: 0.8 % agarose gel showing 5 $\mu$l each of a 200 $\mu$l elution volume of isolated nucleic acids of experiment 8. (a) from 1 million HeLa cells, (b) from 6 million HeLa cells.

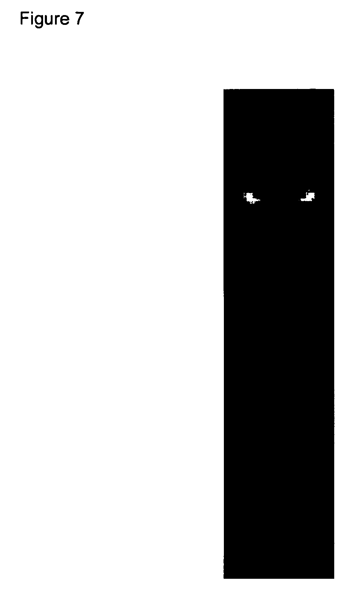

Figure 7: 0.8 % agarose gel showing 5 $\mu$l of a 200 $\mu$l elution volume of isolated nucleic acids at high pH according to experiment 9.

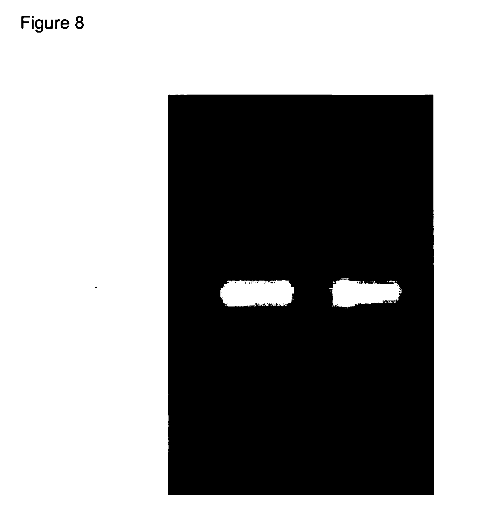

Figure 8: 0.8 % agarose gel showing 5 $\mu$l of a 200 $\mu$l elution volume of isolated nucleic acids according to experiment 10 including a DNA digestion step.

Examples

The following examples provide non-limiting experiments.

Material used:

<u>Solid phase.</u> Magnetic silica particles (MagAttract Suspension B, QIAGEN GmbH), or silica membranes (RNe-asy, QIAGEN GmbH).

<u>Magnet.</u> Manual 10 rack magnet from QIAGEN GmbH (GenoVision).

<u>Lysis and binding solution.</u> 720 g GuHCl (Sigma) wad added to 440 ml of a 0.1 M KH-phthalate solution (Sigma). pH was adjusted to 2.5 with concentrated HCl.

<u>Washing solution I.</u> 720 g GuHCl (Sigma) was added to 440 ml of water (total 7M).

<u>Washing solution II.</u> RNAse free water.

<u>Washing solution III.</u> RNAse free 10 mM Tris, pH 8.

<u>Elution solution.</u> RNAse free 10 mM Tris, pH 8.

<u>Polymer Solution.</u> Typically, 10% (w/v) of a poly(acrylic acid), or its corresponding salt, was dissolved in water, solution adjusted to pH 2.5 with concentrated HCl.

<u>Experiment 1. Variable sample input</u>

General procedure. Cultured HeLa cells were collected (pelleted) from the growth media and frozen in liquid $N_2$. One to 30 million cells were then lysed in 600 $\mu$l of the lysis and binding solution, in a 1.5 ml Eppendorf tube. To this lysate 100 $\mu$l of the 10% (w/v) polymer solution (Mw 2.100, in form of the Na salt, Fluka) were added. After a fast vortex mixing, magnetic silica beads (ca 10 mg, 40 $\mu$l suspension, QIAGEN MagAttract Suspension B) were added. After a binding period of max 10 sec under smooth shaking (this binding time is applicable to all experiments except 4B), the beads were collected by means of a magnet, and the lysate supernatant was discharged. The beads were then resuspended in 600 $\mu$l of washing solution I, resuspended and again collected by means of a magnet. After repeated procedure using 800 $\mu$l of washing solution II and III, a final elution (in 500 $\mu$l, 65°C for 2 min) resulted in isolated nucleic acids.

The whole procedure was easily done within less than 5 min.

The yield of isolated nucleic acids (Figure 1) was measured on a Spectrophotometer (Perkin Elmer, lambda EZ 201, with the 260/280 ratio ranging $\pm$ 2.1, indicating a mixture of RNA and DNA, for gel picture, see experiment 2). As can be seen, the yield is linear referring to sample input in the high sample input load area. RT PCR performance using the elute from 30 million cells (Figure 2) was investigated on a Corbet Real-Time Machine, employing a QIAGEN B-actin RT PCR kit. As can be seen from Figure 2, steady state RT PCR performance emerge (Ct within $\pm$ 13 cycles).

Neither clogging, nor clumping was observed at the solid silica particles in the lysate, and the washing steps and the elution also performed smoothly.

This experiment shows that the invention addresses and solves issues related to disruption of sample, high sample input content, solid phase induced clumping/clogging and binding time, which are disadvantages of the prior art (see experiment 4A).

<u>Experiment 2. Variable polymer weights</u>

6 million HeLa cells were lysed as described in Experiment 1. Poly(acrylic acid) of molecular weight $M_w$ (a) 15.000 (Fluka, as Na salt, adjusted to pH 2.5 with concentrated HCl) (b) 2.100 (Fluka, as Na salt, adjusted to pH 2.5 with concentrated HCl) and (c) 200.000 (Fluka, as free acid, no pH adjustment) was utilized. After procedure as described in experiment 1, the yield was 62 $\mu$g (a), 66 $\mu$g (b), and 42 $\mu$g (c) RNA. The agarose gel performance is shown in Figure 3 (line a, b, and c, respectively). On the gel, high quality RNA is visible, contaminated with DNA. In a real time PCR (Figure 4), amplified RNA (b-acin, QIAGEN QuantiTect Kit) is seen. The RT PCR Ct values were as follows: 12.9 (a), 12.1 (b), and 13.3 (c).

A polymer of Mw 2.100 comprises not more than ca 25 acrylic acid monomer blocks. Testing acetic acid (a monomer), however, did not give any improved effect, compared to the current art.

These experiments show that the method of this invention can be carried out with a broad range of polymer lengths, wherein the monomers are not preferred.

<u>Experiment 3. Different concentrations of polymers</u>

Experiment 2 was repeated with the Mw 2.100 poly(acrylic acid) Na salt polymer. To the lysate 20, 50, and 200 $\mu$l of the 10% acidified (pH 2.5) polymer solution, respectively, were added. The yield was 70, 77 and 63 $\mu$g, respectively. See Figure 5, enclosed gel, line 1-3 represent 20, 50 and 200 $\mu$l of the polymer solution.

This experiment shows that the method of this invention can be carried out with a broad range in respect to concentrations of polymers.

As a control, the 6 million cell lysate was physically disrupted with a syringe (23G) and then 50 $\mu$l of the polymer of weight 2.100, as described in Experiment 3 were added. The performance and yield (80 $\mu$g) were similar to the results of the experiment wherein no physical sample disruption was undertaken.

These experiments show that the method of this invention can be carried out within a broad range in respect to concentrations of polymers. And they unveil the polymers to be preferred embodiments of this invention, even when samples are pre disrupted. This means that disadvantages of the prior art, excluding the disruption part, are still addressed (i.e., binding times, low yield of RNA per sample and per solid phase).

Experiment 4. Prior art methodology

A: As a control experiment of prior art methodology, the same amount of 6 million HeLa cells were lysed in 600 $\mu$l of the lysis and binding solution, but to which no polymer was added. In this case, severe clumping of the silica particles in the lysate and in the subsequent washing steps occurred. Not only low yield (20 $\mu$g), but also contamination with protein was observed (low 260/280 OD ratios).

B: 6 million HeLa cells were lysed under neutral guanidinium conditions, as described in the QIAGEN MagAttract RNA Cell Kit Handbook (QIAGEN GmbH). Binding time here is minimal 2 min. This experiment showed severe clumping of the solid phase in the lysate and in the subsequent washing steps. The clumping was very pronounced especially when EtOH was added to the lysate, and resulted in 22 $\mu$g nucleic acid.

These experiments unveil some of the disadvantages of the prior art methodology.

Experiment 5. Detergent in lysate in the presence or absence of polymers.

12 million HeLa cells were lysed in 1200 $\mu$l of the lysis and binding solution, as described in Experiment 1. To 600 $\mu$l of this lysate were then added 50 $\mu$l of 5% TriX-100, pH 2.5 (sample a), and 50 $\mu$l of a 5% poly(acrylic acid) Mw 2.100, pH 2.5 (sample b). Binding, washing steps and elution were carried out as described in Experiment 1, performed for both samples in parallel. In sample a, the binding and washing steps could be carried out a bit more smoothly compared to experiment 4A, but still worse compared to the preferred embodiment of this invention (Experiment 1). Foaming during the pipetting steps also made handling of sample less convenient. Performance of sample b was comparable to Experiment 1, except in respect of foaming of the sample. The yield was 40 $\mu$g (sample a, 44%) and 90 $\mu$g (sample b).

This experiment shows the presence of a detergent to be applicable in the method of this invention. It also reveals the disadvantages of prior art, when the polymer is absent.

Experiment 6. Different amounts of beads.

Experiment 2 was repeated. 4 million cells were lysed and to the lysate 100 $\mu$l of a 10% solution of Mw 2.100 poly(acrylic acid) polymer were added. The amount of beads was varied between 10 and 80 $\mu$l of MagAttract Suspension B (QIAGEN GmbH), corresponding to between 2.5mg and 20 mg. The yield of nucleic acids after procedure was virtually unaffected at $\pm$40 $\mu$g.

This experiment shows this invention to address and solve issues related to increased solid phase (= bead) consumption, one of the major disadvantages of the prior art.

Experiment 7. Isolation on a membrane.

Experiment 2 was repeated with 3 million HeLa cells. 600 $\mu$l of the lysate were added to 100 $\mu$l of a 10% solution of Mw 15.000 poly(acrylic acid). The lysate was transferred to a QIAGEN RNeasy silica membrane column and processed according to the manufacturer's instructions, however, with the buffers described in the present invention. Final elution was made with pre-heated 200 $\mu$l 10mM Tris pH 8. The yield was 26 $\mu$g.

This experiment shows that the method of the present invention can be used on non magnetic, silica surfaces.

Experiment 8. Addition of the polymer as a Na-salt.

To a lysate prepared as described under experiment 1, but with 1 and 6 mill HeLa cells, 100 $\mu$l of a 10% solution of Poly(acrylic acid) MW 15.000 as a Na-salt (pH ca 7.5) were added. The resulting pH of the lysate was then > 6. After finishing the procedure, on the gel it is clearly visible that the yield of RNA strongly suffers. Only DNA can be seen on the gel (Figure 6). The yield of nucleic acids (= DNA) was about 1 and 6$\mu$g, respectively.

This experiment shows that the method of the present invention highly preferable should be carried out with an acidified lysate.

Experiment 9. Elution at high pH.

A Experiment 2 was repeated with the Mw 2.100 poly(acrylic acid) polymer. Elution was now made with 10 mM Tris, pH 11.5 (pH adjusted from 8 to 11.5 with 3.5M KOH). Elution was performed with 500 $\mu$l, and no heat was involved. The experiment gave ca 25 $\mu$g nucleic acids. But as seen from the gel (Figure 7), no output of intact RNA was observed. As a control, cold elution with elution buffer at pH 8 (10 mM Tris) gives much less output of nucleic acids.
This experiment provides high pH as an alternative to heat elution.
B Experiment 2a was repeated with 3 million HeLa cells. The elution (65°C, 2 min) was performed with 100 $\mu$l of a mixture of 10mM Tris HCl/Base at pH 8.0, 9.0, and 9.7 (25°C), respectively. The yield of nucleic acids increased with pH and was 16, 22 and 24$\mu$g, respectively.
These experiments point towards more efficient (heat) elution with a high pH of the elution buffer.

Experiment 10. Including a DNA digestion step.

3 million HeLa cells were lysed and 50 $\mu$l of the poly(acrylic acid) Mw 2.100 were added and then contacted with silica magnetic beads, as described in Experiment 1. After washing of the beads with wash solution I and III, the magnetic silica beads were redispersed in 200 $\mu$l of a DNA digestion solution (Buffer RDD, QIAGEN GmbH), thereafter 10 $\mu$l of DNAse (out of 1500 k units in 550 $\mu$l water)(QIAGEN GmbH) was added. After a silent incubation period of 2 min, 600 $\mu$l of washing solution I was added to this DNA digest solution. After a further incubation period for 1 min the beads were collected by means of a magnet, supernatant was discharged, and the beads were washed with washing solution II and III as described in Experiment 1. The yield of RNA was 19 $\mu$g. The OD reading ratio 260/280 was now 2.18, indicating less DNA contamination in this case (compared to 2.1 in Experiment 1). View on enclosed gel (Figure 8), clearly points towards full removal of genomic DNA (see Figure 3, gel after Experiment 2 as a reference in which DNA is present).

**Claims**

1. A process for isolating nucleic acids from a nucleic acid containing sample, comprising contacting a nucleic acid containing acidified lysate with a nucleic acid binding solid surface, wherein the lysate comprises further at least the following components

    a) a chaotropic salt,
    b) an organic, water soluble polymer.

2. A process according to claim 1, further comprising at least one of the following additional step(s): washing, purification, nuclease treatment (optionally) and elution(s)

3. A process according to claim 1 or 2, wherein the polymer comprises acidic chain groups or acidic side groups, or a combination thereof, provided as free acid(s), as corresponding salt(s) or as a combination thereof.

4. A process according to any of claims 1 to 3, wherein the polymer comprises any acid polymers with carboxylic, acrylic, methacrylic, sulfonic and/or phosphoric acid groups.

5. A process according to claim 4, wherein the polymer comprises poly (acrylic acids) containing at least 3 monomers or more and preferably having molecular weights ranging from Mw 2.000 to 200.000.

6. A process according to any of claims 1 to 5, wherein the polymer is added with a final concentration in the lysate ranging from $1 \times 10^{-5}$ to less than 50%, more preferably from 0.05 to 5%, most preferably in the range of 0.1 to 2.5%.

7. A process according to any of claims 1 to 6, wherein the pH of the overall lysate is from 1 to 6, more preferably from 2 to 4, most preferably from 2 to 3.

8. A process according to any of claims 1 to 7, wherein the lysate additionally comprises a detergent, wherein the detergent is added either

    i) to the chaotropic lysis solution prior to contacting said solution with the nucleic acid containing sample; or
    ii) to the lysate after lysis of the nucleic acid containing sample.

**9.** A process according to any of claims 1 to 8, wherein the chaotropic salt comprises a guanidinium salt, urea, or sodium or potassium iodide, sodium or potassium chlorate, sodium or potassium perchlorate or sodium or potassium (iso)thiocyanate or any mixtures thereof.

**10.** A process according to any of claims 1 to 9, wherein the water soluble acid polymer is added either

    i) to the chaotropic lysis solution prior to contacting said solution with the nucleic acid containing sample; or
    ii) to the lysate after lysis of the nucleic acid containing sample, but prior to introduction of the solid surface.

**11.** A process according to any of claims 1 to 10, wherein the nucleic acid-binding solid surface comprises sheets, sieves, sinters, webs, membranes, beads and fibres, silica surfaces, non magnetic silica beads and/or magnetic silica beads.

**12.** A process according to any of claims 1 to 11, wherein the nucleic acid containing sample comprises rRNA, mRNA, total RNA or DNA, or a combination thereof.

**13.** A kit for carrying out the process of any of claims 1 to 12, which kit comprises:

    (a) a chaotropic salt;
    (b) a water soluble, acid organic polymer; and
    (c) a nucleic acid binding solid phase capable of binding nucleic acid in the presence of the chaotrope,

wherein (a) and (b) can independently either be provided as a powder or as a solution, preferably as an aqueous solution, and (c) can be provided as dry solid material or as dispersion.

**14.** A kit according to claim 13, further comprising buffers and/or solutions applicable to carry out at least one of the additional steps mentioned in claim 2.

Figure 1

Figure 2

Figure 3

Figure 4

Figure 5

Figure 6

Figure 7

Figure 8

| European Patent Office | EUROPEAN SEARCH REPORT | Application Number EP 06 01 3576 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/01359 A (QIAGEN GMBH; COLPAN, METIN; SCHORR, JOACHIM; HERMANN, RALF; FEUSER, PE) 12 January 1995 (1995-01-12) | 1-4,6-14 | INV. C12N15/10 |
| Y | * the whole document * | 5 | |
| Y | WO 03/091452 A (QIAGEN AS; DEGGERDAL, ARNE; SKAGESTAD, VIDAR) 6 November 2003 (2003-11-06) * the whole document * | 1-14 | |
| Y | LIN ET AL: "Preparation and properties of poly(acrylic acid) oligomer stabilized superparamagnetic ferrofluid" JOURNAL OF COLLOID AND INTERFACE SCIENCE, ACADEMIC PRESS, NEW YORK, NY, US, vol. 291, no. 2, 15 November 2005 (2005-11-15), pages 411-420, XP005100711 ISSN: 0021-9797 * the whole document * | 1-14 | |
| A | ZHANG C ET AL: "Lysozyme purification from tobacco extract by polyelectrolyte precipitation" JOURNAL OF CHROMATOGRAPHY, ELSEVIER SCIENCE PUBLISHERS B.V. AMSTERDAM, NL, vol. 1069, no. 1, 25 March 2005 (2005-03-25), pages 107-112, XP004790340 ISSN: 0021-9673 | | TECHNICAL FIELDS SEARCHED (IPC) C12N |

The present search report has been drawn up for all claims

| Place of search Munich | Date of completion of the search 7 September 2006 | Examiner Heder, Andreas |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**EP 1 873 241 A1**

## ANNEX TO THE EUROPEAN SEARCH REPORT
## ON EUROPEAN PATENT APPLICATION NO.

EP 06 01 3576

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2006

| Patent document cited in search report | | Publication date | Patent family member(s) | | Publication date |
|---|---|---|---|---|---|
| WO 9501359 | A | 12-01-1995 | AT | 200293 T | 15-04-2001 |
| | | | CA | 2142910 A1 | 12-01-1995 |
| | | | DE | 4321904 A1 | 12-01-1995 |
| | | | DK | 658164 T3 | 18-06-2001 |
| | | | EP | 0658164 A1 | 21-06-1995 |
| | | | ES | 2155477 T3 | 16-05-2001 |
| | | | JP | 8501321 T | 13-02-1996 |
| | | | PT | 658164 T | 28-09-2001 |
| | | | US | 6383393 B1 | 07-05-2002 |
| WO 03091452 | A | 06-11-2003 | AU | 2003219396 A1 | 10-11-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5681946 A
- US 5234809 A
- US 6027945 A
- US 6582922 B
- WO 0384976 A
- WO 9501359 A
- US 5973137 A
- US 5155018 A
- US 4843155 A
- US 5990302 A
- WO 9618731 A
- WO 03084976 A

**Non-patent literature cited in the description**

- **NOONBERG.** *BioTechniques,* 1995, vol. 19, 731-733